Europäisches Patentamt

European Patent Office

Office européen des brevets

(11) Publication number: **0 210 774**
**A1**

(12) **EUROPEAN PATENT APPLICATION**

(21) Application number: **86305353.4**

(22) Date of filing: **11.07.86**

(51) Int. Cl.⁴: **A 61 K 7/08**
**A 61 K 7/06**

(30) Priority: **11.07.85 JP 153714/85**

(43) Date of publication of application:
**04.02.87 Bulletin 87/6**

(84) Designated Contracting States:
**CH DE FR LI**

(71) Applicant: **SHIONOGI & CO., LTD.**
**12, Dosho-machi 3-chome Higashi-ku**
**Osaka 541(JP)**

(72) Inventor: **Ukita, Hisako**
**5-10-7, Uematsu-cho**
**Yao-shi Osaka(JP)**

(72) Inventor: **Kawai, Sadao**
**1-15-7, Kawai**
**Matsubara-shi Osaka(JP)**

(72) Inventor: **Egawa, Shohei**
**2-4-7, Nanatsumatsu-cho**
**Amagasaki-shi Hyogo(JP)**

(74) Representative: **Bizley, Richard Edward et al,**
**BOULT, WADE & TENNANT 27 Furnival Street**
**London EC4A 1PQ(GB)**

(54) **Anti-dandruff shampoo.**

(57) An anti-dandruff shampoo comprising
(a) metal salts of bis(2-pyridylthio-1-oxide), (b) betaine,
(c) triethanolamine alkyl sulfate and alkali metal alkyl sulfate
and (d) fatty acid alkylolamide; enables the metal salt of bis(2-
pyridylthio-1-oxide) to be stably dispersed over a long period
of time.

EP 0 210 774 A1

## ANTI-DANDRUFF SHAMPOO

The present invention relates to anti-dandruff shampoos.

Most anti-dandruff shampoos comprise shampoo components blended with a pyrithione having antimicrobial activity, such as metal salts of bis (2-pyridylthio-1-oxide), preferably zinc bis (2-pyridylthio-1-oxide) (generic name: zinc pyrithion, hereinafter abbreviated as ZP). These substances, however, are generally water insoluble or have a high specific gravity, and thus they are difficult to stably disperse in the shampoo over a long period. When stored for long periods, the antimicrobial substances precipitates leading to marked quality deterioration.

To prevent such precipitation, the addition of various dispersants has been investigated: for example, addition of various cellulose derivatives and water soluble polyacrylic acid, addition of magnesium lauryl ether sulfate (JPN unexam. Patent Publication No. 52-12207), addition of hydrogenated castor oil (JPN unexam. Pat. Pub. No. 56-62899), and addition of alkanolamides of higher fatty acid and mono- or di-higher fatty acid esters of ethylene glycol or polyethylene glycol (JPN unexam. Pat. Pub. No. 58-29900) has been proposed. Though some effects have been obtained by these investigations, there still remain such disadvantages that stabilization over a long period is insufficient or substances unnecessary for the primary purpose of shampoo are used.

According to the present invention there are provided anti-dandruff shampoos comprising the following components:

    a.  metal salt of bis(2-pyridylthio-1-oxide),

b.  betaine,

c.  triethanolamine alkyl sulfate and alkali
    metal alkyl sulfate, and

d.  fatty acid alkylolamide.

The present invention sets out to resolve the afore-mentioned problems by the discovery that by appropriate addition of betaine, triethanolamine alkyl sulfate and alkali metal alkyl sulfate, and fatty acid alkylolamide, pyrithion, particularly metal salts of bis(2-pyridylthio-1-oxide) can be stably dispersed in the shampoo over a long period of time.

The shampoos of the present invention may be prepared by using as essential components, in preferred quantities, (a) a metal salt of bis(2-pyridylthio-1-oxide), 0.01 - 3.0%, most preferably 0.01 - 1.0%, (b) betaine 4.6 - 10%, most preferably 6.5 - 10%, (c) triethanolamine alkyl sulfate and alkali metal alkyl sulfate, 3.4 - 16%, most preferably 6.5 - 10%, and (d) fatty acid alkylolamide, 4 - 25%, most preferably 5.2 - 25%.

Unless otherwise specified, the amounts of additives are represented as weight % (w/wt%).

The betaine presently used, which has the properties of an amphoteric surfactant, includes alkylsulfobetaine or alkylaminoacetic acid betaine. Alkylsulfobetaine may be cocoyl dimethylsulfopropyl ammonium betaine (CFTA: Cocosultaine) or coco fatty acid amidopropyldimethyl hydroxysulfopropyl ammonium betaine. Alkylaminoacetic acid betaine may be lauryl dimethylaminoacetic acid betaine, decyl dimethylaminoacetic acid betaine, cocyl dimethylaminoacetic acid betaine (CFTA: Cocobetaine), and coco fatty acid amidopropyldimethyl aminoacetic acid betaine. "CFTA" indicates the designation described in the CFTA Cosmetic Ingredient Dictionary published by the Cosmetic Toiletry and Fragrance Association in the U.S.A.

Triethanolamine alkylsulfate and alkali metal alkyl sulfate are types of anion surfactants and may be used as a mixture. The alkali metal alkyl sulfate is preferably added in an amount of about 3% based on the total amount of the composition.

An example of triethanolamine alkylsulfate is triethanolamine lauryl sulfate. The alkali metal alkyl sulfate is exemplified by sodium lauryl sulfate, potassium lauryl sulfate, sodium myristyl sulfate, sodium cetyl sulfate, sodium stearyl sulfate, and sodium hydrogenated coco fatty acid monoglyceride sulfate. If desired, a polyoxyethylene (hereinafter abbreviated as POE) alkyl ether sulfate such as sodium POE lauryl ether sulfate, triethanolamine POE lauryl ether sulfate, sodium POE alkyl ether sulfate, triethanolamine POE alkyl ether sulfate, sodium POE nonylphenyl ether sulfate or triethanolamine POE nonylphenyl ether sulfate, and/or a N-acylamino acid surfactant, such as sodium N-lauroyl sarcosinate, may be added.

Fatty acid alkylolamide acts as a non-ionic surfactant, and is a reaction product of a $C_{12}$-$C_{20}$ fatty acid or its methyl ester with monoethanolamine, diethanolamine, or isopropanolamine in molar ratio of 1:1 (to produce a 1:1 type) or 1:2 (to produce a 1:2 type). Examples of 1:1 type and 1:2 type fatty acid alkylolamide are coco fatty acid monoethanolamide, stearoyl monoethanolamide, lauroyl monisopropanolamide, coco fatty acid diethanolamide, lauroyl diethanolamide, myristeoyl diethanolamide, stearoyl diethanolamide, and oleoyl diethanolamide. If desired, other non-ionic POE sorbitan fatty acid ester surfactants, such as POE sorbitan monooleate and POE sorbitan monostearate, amy be added.

The optimum compound ratio of the essential components (b) betaine (c) triethanolamine alkyl sulfate and alkali metal alkyl sulfate, and (d) fatty acid alkylolamide is in the weight ratios of b:c:d = 1:(0.75 - 1.6):(0.8 - 3.0), preferably, b:c:d = (8 -8.5):(8 - 8.5): (8.13.5).

In addition to the above essential components, a water-soluble polymer cationized with quaternary nitrogen groups (hereinafter called a cationic copolymer) can optionally can be added as a finisher to leave the hair soft and moist. The polymer may be added in a range from 0.1 to 3 %, preferably about 0.5%. Available cationic copolymers include, for example, quaternary ammonium cellulose ether derivatives, quaternary ammonium poly(trialkyl-aminoethyl) methacrylate derivatives, and water-soluble cationised copolymers of tetraethylene pentamine and epichlorohydrin.

Examples of the fat-restoring additives which may be added include isopropyl palmitate (hereinafter abbreviated as IPP), butyl stearate, silicone oil, lanolin, octyldodecyl myristate, myristyl myristate, cetyl lactate, polyethylene glycol, and cotyl dodecanol.

Examples of humectants which may be added include propylene glycol, glycerin, and polyhydric alcohols.

Ordinary preservatives, hair-grooming agents, perfumes, coloring agents and pearl agents can be added if desired. Particularly preferred are those described in official documents such as Cosmetics Raw Material Standards. Suitable preservatives include benzoid acid, benzoates, salicylic acid, salicylates, and parahydroxybenzoic acid esters.

A pH-adjustor may be used in order that the final pH value of the shampoo is in a range of 6.5 to 8, preferably about 7.0. Preferable pH-adjustors include, for example, inorganic or organic bases such as sodium hydroxide, triethanolamine, and diethanolamine or inorganic or organic acids such as hydrochloric acid and citric acid.

The shampoo composition according to the invention may be prepared by generally accepted methods. For example, the betaine and anionic surfactant may be dissolved in an adequate quantity of water at elevated temperature, and the non-ionic surfactant and a pearl agent, if desired, mixed therein. To the mixture, if desired, are added and well mixed a fat-restoring additive, a preservative, and a humectant dissolved in a small amount of water with heating. A hair grooming agent, perfume, colouring agent, and others may be further admixed as required. After lowering temperature of the mixture, the mixture is adjusted to pH 6.5 - 8 with a pH-adjustor, and the total volume is made up to 100% by the addition of water.

The following Examples and Experiments illustrate the compositions of the invention, but are not intended to limit the scope thereof.

<u>Examples 1 to 22</u>

A.    Amphoteric surfactant
      Anionic surfactant
      Deionized water

B.    Nonionic surfactant
      Pearl Agent

C.    Cationic copolymer
      Fat-restoring additive
      Humectant

D.    Hair-grooming agent

- 6 -

Perfume

Coloring agent

pH-adjustor Deionized water

E. Metal salt of bis(2-pyridylthio-1-oxide)

B is added into A dissolved under heat at 50°C, and the mixture is stirred for 10 min at 50°C (or at 75°C if pearl agent is added). Then, C is added thereto and kept at 50°C for 10 min, and while stirring and with the addition of D the mixture is slowly cooled down to 30°C. The mixture is slowly blended into the finely powdered E, adjusted to pH 6.5 - 8, and the total volume made up to 100% with the addition of deionized water.

According to this procedure, the composition in Examples 1 through 22 were prepared (Table 1).

All the quantities of the surfactants used in the invention were represented on the basis of 100% active ingredients.

Table 1 (No. 2)

| Purpose | Component / Example No. | 13 | 14 | 15 | 16 | 17 | 18 | 19 | 20 | 21 | 22 |
|---|---|---|---|---|---|---|---|---|---|---|---|
| Antidandruff | Zinc pyrithione (ZP) | 0.4 | 0 4 | 0 4 | 0 4 | 0 4 | 0 4 | 0.4 | 0.4 | 0.5 | 1 |
| Film-forming Substance | Cationic Copolymer | 0.5 | 0.5 | 0.5 | 0.5 | 0 5 | 0.5 | 0.5 | 0.5 | | |
| Amphoteric Surfactant | Lauryldimethylaminocarboxy-methylbetaine | 8 | 8 | 8 | 8 | | | | | 7 | 7 |
| | Coco fatty acid amidopropyl-dimethyl aminoacetic acid betaine | | | | | 4.5 | 4.5 | | | | |
| | Coco fatty acid amidopropyl-dimethyl hydroxysulfopropyl ammonium betaine | | | | | | | 4.5 | 4.5 | | |
| Anionic Surfactant | Triethanolamine lauryl sulfate | 5 | 5 | 5 | 5 | 1.5 | 1.5 | 1.5 | 1.5 | 3.2 | 3.2 |
| | Sodium lauryl sulfate | 3 | 3 | 3 | 3 | 3 | 3 | 3 | 3 | 3 | 3 |
| | Sodium cetyl sulfate | | | 5 | 3 | 3 | | | | | |
| | Potassium lauryl sulfate | | | | | | 3 | | | | |
| | Sodium stearyl sulfate | 3 | | | | | | | | | |
| | Sodium POE alkyl sulfate | | | | | | | 3 | 3 | | |
| | Triethanolamine POE alkyl ether sulfate | | | | | | | | 3 | | |
| | Sodium POE lauryl ether sulfate | | 1.5 | | | | | | | | |
| | Sodium N-lauroyl sarcosinate | | | | | | | | | 1.0 | 1.0 |
| Nonionic Surfactant | Laurylsulfodiethanolamide | 10 | 10 | 10 | 8 | 4.5 | 4.5 | 4.5 | 4.5 | 21 | 15 |
| | POE sorbitan monooleate | | | | | | | | | | |
| | POE sorbitan monostearate | | | | | | | | | | |
| Others | Fat-restoring additives, color additives, perfumes, and the like | An appropriate amount ||||||||||
| pH-adjustor | Citric acid (0.1% or more) | 0.1 | 0.1 | 0.1 | 0.1 | 0.1 | 0.1 | 0.1 | 0.1 | 0.5 | 0.5 |
| | Deionized water | A necessary amount to make the whole 100% ||||||||||

Table 1

| Purpose | Component / Example No. | 1 | 2 | 3 | 4 | 5 | 6 | 7 | 8 | 9 | 10 | 11 | 12 |
|---|---|---|---|---|---|---|---|---|---|---|---|---|---|
| Antidandruff | Zinc pyrithione (ZP) | 0.4 | 0.4 | 0.4 | 0.4 | 0.4 | 0.4 | 0.4 | 0.4 | 0.4 | 0.4 | 0.4 | 0.4 |
| Film-forming Substance | Cationic Copolymer | | | | | 0.5 | 0.5 | 0.5 | 0.5 | 0.5 | 0.5 | 0.5 | 0.5 |
| Amphoteric Surfactant | Lauryldimethylaminocarboxy-methylbetaine | 6.5 | 8 | 8.5 | 8.5 | 4.6 | 8 | 10 | 10 | 10 | 8 | 8 | 8 |
| | Coco fatty acid amidopropyl-dimethyl aminoacetic acid betaine | | | | | | | | | | | | |
| | Coco fatty acid amidopropyl-dimethyl hydroxysulfopropyl ammonium betaine | | | | | | | | | | | | |
| Anionic Surfactant | Triethanolamine lauryl sulfate | 3.5 | 5 | 5.5 | 5.5 | 1.5 | 5 | 7 | 7 | 7 | 5 | 5 | 5 |
| | Sodium lauryl sulfate | 3 | 3 | 3 | 3 | 3 | 3 | 3 | 3 | 3 | 3 | 3 | 3 |
| | Sodium cetyl sulfate | | | | | | | | | | 1 | | |
| | Potassium lauryl sulfate | | | | | 3 | | | | | | 3 | |
| | Sodium stearyl sulfate | | | | | | | | | | | | |
| | Sodium POE alkyl sulfate | | | | | | | | | | | | 5 |
| | Triethanolamine POE alkyl ether sulfate | | | | | | | | | | | | |
| | Sodium POE lauryl ether sulfate | | | | | | | | | | | | |
| | Sodium N-lauroyl sarcosinate | | | | | | | | | | | | |
| Nonionic Surfactant | Laurylsulfodiethanolamide | 6.5 | 10 | 13.5 | 8.5 | 4.5 | 10 | 10 | 10 | 25 | 10 | 10 | 10 |
| | POE sorbitan monooleate | | | | | | | | 2 | | | | |
| | POE sorbitan monostearate | | | | | | | | | 2 | | | |
| Others | Fat-restoring additive, color additives, perfumes, and the like | An appropriate amount | | | | | | | | | | | |
| pH-adjustor | Citric acid (0.1% or more) | 0.3 | 0.3 | 0.3 | 0.3 | 0.1 | 0.1 | 0.1 | 0.1 | 0.1 | 0.1 | 0.1 | 0.1 |
| | Deionized water | A necessary amount to make the whole 100% | | | | | | | | | | | |

- 7 -

## Experiment 1

A paired comparison test of the general characteristics of the invention was performed with commercially available compositions (hereinafter abbreviated as CACs). Characteristics chosen were:

1. Bubbling

2. Detergency

3. Rinsability

4. Softness of hair (after washing)

5. Brushability

6. Dandruff

7. Itching

8. Overall evaluation

Volunteers were asked to answer the questionnaire, with several persons assigned to each item. The overall evaluation results of the questionnaire are shown in Table 2.

Table 2

| Composition Tested | | Number of Volunteers answered "Superior" | | Number of Volunteers |
|---|---|---|---|---|
| Invention | CAC | Invention | CAC | |
| Example 21 | CAC A | 4*1 | 4*1 | 10.5*1(2.5*1)*2 |
| Example 22 | CAC A | 6 | 4 | 10 |

*1: Means on 2 tests.

*2: Numeral surrounded by the parenthesis shows how many volunteers answered "No difference".

- 11 -

As obvious from the test results, there was no significant difference in general impressions in usage over CACs.

Experiment 2

The stability (dispersibility) of the compositions according to the invention was examined by the appearance change with time. (Table 3)

In Table 3, (◎) denotes "no change", (○) "hardly changed", and (△) "slight unevenness".

By those tests above, it was verified that the compositions according to the invention are anti-dandruff shampoos of excellent commercial value having exceptionally qualities of feel and a long shelf life.

It can be seen from the foregoing that the antimicrobial agent can be suspended in the shampoo in a stable state over a long period of time to provide compositions having a uniform quality and durable anti-dandruff effect, as well as a pleasant feel which markedly increases the commercial value of the shampoo.

The invention further provides for the use of a mixture of (a) betaine (b) triethanolamine alkyl sulfate and alkali metal alkyl sulfate and (c) fatty acid alkylolamide in the stabilisation of a dispersion of a metal salt of bis(2-pyridylthio-1-oxide). The invention still further provides a method of conditioning hair solely for cosmetic improvement which comprises applying thereto a shampoo as hereinbefore described.

Table 3

| Conditions of the test | | Example No. | 1 | 2 | 3 | 4 | 5 | 6 | 7 | 8 | 9 | 10 | 11 | 12 | 13 | 14 | 15 | 16 | 17 | 18 | 19 | 20 | 21 | 22 |
|---|---|---|---|---|---|---|---|---|---|---|---|---|---|---|---|---|---|---|---|---|---|---|---|---|
| Temp. | Term | Change | | | | | | | | | | | | | | | | | | | | | | |
| 45°C | After 3 Months | Appearance | ○ | ○ | ○ | ○ | ◎ | ◎ | ◎ | ◎ | ◎ | ◎ | △ | ◎ | ○ | ○ | ○ | ◎ | △ | △ | △ | △ | ○ | ○ |
| | | Precipitate | None | | | | | | | | | | | | | | | | | | | | | |
| | After 4 Months | Appearance | △ | △ | △ | △ | ○ | ○ | ○ | ○ | ○ | ○ | | | | | | | | | | | | |
| | | Precipitate | None | | | | | | | | | | | | | | | | | | | | | |
| | After 5 Months | Appearance | | | | | ○ | ○ | ○ | ○ | ○ | ○ | | | | | | | | | | | | |
| | | Precipitate | | | | | None | | | | | | | | | | | | | | | | | |

## CLAIMS

1.  An anti-dandruff shampoo comprising
(a) metal salt of bis(2-pyridylthio-1-oxide),
(b) betaine
(c) triethanolamine alkyl sulfate and alkali metal alkyl sulfate, and
(d) fatty acid alkylolamide.

2.  A shampoo as claimed in claim 1 wherein the metal salt of bis(2-pyridylthio-1-oxide) is the zinc salt.

3.  A shampoo as claimed in claim 1 or claim 2 wherein the betaine is an alkylaminoacetic acid betaine or an alkylsulfobetaine.

4.  A shampoo as claimed in any one of claims 1 to 3 comprising
(a) metal salt of bis(2-pyridylthio-1-oxide) 0.01-1.0%
(b) betaine                                   4.6 - 10%
(c) triethanolamine alkyl sulfate and alkali metal alkyl sulfate                         3.4 - 16%
(d) fatty acid alkylolamide                   4   - 25%

5.  A shampoo as claimed in any one of claims 1 to 3 comprising;
(a) metal salt of bis(2-pyridylthio-1-oxide) 0.01-1.0%
(b) betaine                                   6.5 - 10%
(c) triethanolamine alkyl sulfate and alkali metal alkyl sulfate                         6.5 - 10%
(d) fatty acid alkylolamide                   5.2 - 25%

6.  A shampoo as claimed in any one of claims 1 to 5 which further comprises one or more of the following;

cationic copolymers, fat-restoring additives, humetants, ordinary preservatives, hair-grooming agents, perfumes, colouring agents and pearl agents.

7.  The use of a mixture of (a) betaine (b) triethanolamine alkyl sulfate and alkali metal alkyl sulfate and (c) fatty acid alkylolamide in the stabilisation of a dispersion of a metal salt of bis(2-pyridylthio-1-oxide).

8.  A method of conditioning hair solely for cosmetic improvement which comprises applying thereto a shampoo as claimed in any one of claims 1 to 6.

European Patent Office

**EUROPEAN SEARCH REPORT**

Application number

EP 86 30 5353

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int Cl 4) |
| Y | FR-A-2 315 905 (JOHNSON & SON) * Page 2, lines 26-39; page 3, line 38 - page 7, line 20; examples 3,4; page 8, lines 14-20; claims 1-4,7 * | 1-6,8 | A 61 K 7/08 A 61 K 7/06 |
| | --- | | |
| Y | EP-A-0 117 135 (JOHNSON & JOHNSON BABY PRODUCTS) * Whole document * | 1-6,8 | |
| | --- | | |
| Y | US-A-3 957 970 (KORKIS) * Example 2 * | 1-5,7 8 | |
| | --- | | |
| Y | GB-A-2 031 942 (ALBRIGHT & WILSON) * Whole document * | 1-5,7 8 | |
| | --- | | TECHNICAL FIELDS SEARCHED (Int. Cl 4) |
| A | BE-A- 702 495 (PROCTER & GAMBLE) * Claims 1,5,7,8; page 4, line 3 - page 5, line 21; page 11, line 18 - page 12, line 5; page 12, line 28 - page 13, line 9 * | 1-6,8 | A 61 K |
| | --- | | |
| A | FR-A-2 178 819 (PROCTER & GAMBLE) * Whole document * | 1-6,8 | |
| | ----- | | |

The present search report has been drawn up for all claims

| Place of search | Date of completion of the search | Examiner |
|---|---|---|
| THE HAGUE | 22-10-1986 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82